# EUROPEAN PATENT APPLICATION

(11) **EP 4 840 875 A1**
(43) Date of publication of application: **18.11.2026**
(21) Application number: 25914215.6
(22) Date of filing: 21.02.2025
(51) Int. Cl.: C07K 14/245, C12N 1/21, C12P 13/08, C12R 1/19

(54) **MUTANT MNTP PROTEIN AND STRAIN EXPRESSING SAME FOR HIGH-YIELD L-THREONINE PRODUCTION**

(30) Priority: 03.01.2025 CN 202510007767
(71) Applicant: Hulunbeier Northeast Fufeng Biotechnologies Co., Ltd, Hulun Buir, Inner Mongolia 162650 (CN); QIQIHAR LONGJIANG FUFENG BIOTECHNOLOGIES CO., LTD., Qiqihar, Heilongjiang 161031 (CN)
(72) Inventor: WANG, Juncheng, Inner Mongolia 162650 (CN); XU, Zhiying, Inner Mongolia 162650 (CN); FANG, Ning, Inner Mongolia 162650 (CN); LI, Siqi, Inner Mongolia 162650 (CN); SONG, Yu, Inner Mongolia 162650 (CN); LIU, Yuantao, Inner Mongolia 162650 (CN); LIU, Shizhou, Inner Mongolia 162650 (CN); SHI, Shuzhi, Inner Mongolia 162650 (CN); LIU, Chengcai, Qiqihar, Heilongjiang 161031 (CN); ZHAO, Lankun, Qiqihar, Heilongjiang 161031 (CN)
(74) Representative: Tiburzi, Andrea
(86) International application number: PCT/CN2025/078535
(87) International publication number: WO 2026/143819

(57) **Abstract**

Provided in the present invention are a manganese ion transporter (MntP) protein mutant and a recombinant strain expressing the mutant for high-yield L-threonine production. The mutant has an amino acid sequence as shown in SEQ ID NO: 2, which is obtained by means of mutating glycine (G) at position 25 to aspartic acid (D) in a wild-type MntP protein sequence as shown in SEQ ID NO: 1. By means of homologous recombination, the mutant is introduced into an Escherichia coli strain, providing the constructed recombinant strain with an increased yield of L-threonine.

## Description

### TECHNICAL FIELD

The present application belongs to the field of microbial fermentation and specifically relates to a mutant MntP protein and a high-yield L-threonine-producing strain expressing the same.

### BACKGROUND

As one of the eight essential amino acids for the growth of human beings and animals, threonine is an important nutritional enhancer and has been widely used in medicine, chemical reagents, food fortifiers, feed additives, and other fields. Currently, threonine is mainly produced industrially through microbial fermentation, typically by modifying microorganisms to increase the yield of L-threonine. A variety of bacteria can be used in the production of L-threonine, such as mutant strains obtained by induction of wild-type *Escherichia*, *Corynebacterium*, *Brevibacterium*, etc., as production strains. As global demand for threonine continues to increase, the construction and modification of high-yield threonine strains are particularly important. Specific examples include: U.S. Patent US8293505B2 filed in 2006 by Ajinomoto Co., Inc., which utilizes an *Escherichia coli* (*E*. *coli*) MG1655 strain to increase the expression of the pyrE gene encoding orotate phosphoribosyltransferase by increasing the copy number of the pyrE gene; and Chinese Patent CN115572717A filed in 2021 by Meihua Group, which modifies the *E*. *coli* MHZ-0215-2 genome by introducing a point mutation to generate a CreC protein variant R77P, thereby improving threonine production capacity, where the modified R77P mutant strain MHZ-0221-4 achieved an average shake-flask conversion rate of 20.6%, an increase of 4.39 percentage points compared with the starting strain.

The protein encoded by the *mntP* gene is a Mn²⁺ transporter, a key protein that regulates the transport and metabolism of manganese ions (Mn²⁺) in living organisms, and plays an important role in maintaining the level of intracellular manganese. For organisms, manganese ions play a critical role in various biological metabolic processes and are essential nutrients for intracellular activities. Manganese ions serve as cofactors for a variety of enzymes, including arginase, glutamine synthetase (GS), pyruvate carboxylase, and manganese superoxide dismutase (Mn-SOD). These enzymes are important components of the amino acid metabolic pathways in organisms.

However, there has been no literature report to date on increasing the yield of threonine through mutation of the Mn²⁺ transporter.

### SUMMARY

In the present application, whole-genome sequencing is carried out on a mutagenized strain FFTHR-1 with a high threonine yield that exists in the laboratory. It is found that a mutation occurs at position 25 of the amino acid sequence of the MntP manganese ion transporter encoded by the *mntP* gene, specifically a mutation from glycine to aspartic acid, designated as MntP G25D. The mutation is one of the reasons for the high threonine production of the mutagenized strain. Introducing the gene encoding the mutant protein into other production strains can also increase the threonine yield.

In a first aspect, the present application provides a mutant MntP manganese ion transporter, wherein relative to a wild-type MntP manganese ion transporter having a sequence as shown in SEQ ID NO: 1, the mutant MntP manganese ion transporter includes at least the following mutation site: the amino acid at position 25 of the wild-type MntP manganese ion transporter is mutated from glycine to aspartic acid.

In some embodiments, the mutant MntP manganese ion transporter may further contain substitutions, deletions, insertions, additions, or inversions of other amino acids, provided that the mutation does not affect the function of the MntP manganese ion transporter. In a specific embodiment, the amino acid sequence of the mutant MntP manganese ion transporter is as shown in SEQ ID NO: 2.

In some embodiments, the sequence of an *mntP* gene encoding the wild-type MntP manganese ion transporter is as shown in SEQ ID NO: 3. In some embodiments, the sequence of a mutant *mntP* gene encoding the mutant MntP manganese ion transporter is as shown in SEQ ID NO: 4 (designated as the *mntP G25D* gene).

In a second aspect, the present application provides the use of the mutant MntP manganese ion transporter for increasing the threonine yield in a threonine-producing strain.

In a third aspect, the present application provides a high-yield L-threonine-producing recombinant strain which expresses the mutant MntP manganese ion transporter.

In some embodiments, the recombinant strain is *E*. *coli* and is preferably obtained by modification based on an *E*. *coli* THRS strain, an *E*. *coli* THRS-6 strain, an *E*. *coli* THRS-YbiI Q86L mutant strain, or an *E*. *coli* THRS-6-YbiI Q86L mutant strain.

The *E*. *coli* THRS strain is the *E*. *coli* THRS strain involved in Patent CN202411834458.7 and is deposited under the deposit number CGMCC NO. 31694 on August 20, 2024 at the China General Microbiological Culture Collection Center located at NO. 3, NO. 1 West Beichen Road, Chaoyang District, Beijing, China.

The *E*. *coli* THRS-6 strain is the *E*. *coli* THRS-6 strain involved in Patent CN202411834455.3 and is deposited under the deposit number CGMCC NO. 31695 on August 20, 2024 at the China General Microbiological Culture Collection Center located at NO. 3, NO. 1 West Beichen Road, Chaoyang District, Beijing, China.

The THRS-YbiI Q86L mutant strain is an *E*. *coli* THRS strain expressing a mutant YbiI protein, wherein the amino acid sequence of the mutant YbiI protein is as shown in SEQ ID NO: 13, and the mutant YbiI protein is obtained by mutating glutamine at position 86 of a wild-type YbiI protein to leucine. The *ybiI* gene of an *E*. *coli* THRS strain is replaced with a mutant *ybiI* gene to enable the *E*. *coli* THRS strain to express the mutant YbiI protein, and as a result, the THRS-YbiI Q86L mutant strain is obtained.

The THRS-6-YbiI Q86L mutant strain is an *E*. *coli* THRS-6 strain expressing a mutant YbiI protein, wherein the amino acid sequence of the mutant YbiI protein is as shown in SEQ ID NO: 13, and the mutant YbiI protein is obtained by mutating glutamine at position 86 of a wild-type YbiI protein to leucine. The *ybiI* gene of an *E*. *coli* THRS-6 strain is replaced with a mutant *ybiI* gene to enable the *E*. *coli* THRS-6 strain to express the mutant YbiI protein, and as a result, the THRS-YbiI Q86L mutant strain is obtained.

In some embodiments, the nucleotide sequence of the mutant *ybiI* gene is as shown in SEQ ID NO: 14 (designated as the *ybiI Q86L* gene).

In a fourth aspect, the present application provides a method for preparing the recombinant strain described in the third aspect, wherein the method includes the following steps: designing homology arm primers with a gene encoding a mutant MntP manganese ion transporter as a template, constructing a PCR amplification product, transferring the PCR amplification product into a threonine-producing strain, and replacing a target region via homologous recombination to obtain the recombinant strain. The threonine-producing strain is *E*. *coli* and is preferably an *E*. *coli* THRS strain, an *E*. *coli* THRS-6 strain, an *E*. *coli* THRS-YbiI Q86L mutant strain, or an *E*. *coli* THRS-6-YbiI Q86L mutant strain.

In a fifth aspect, the present application provides a method for producing L-threonine, wherein the method includes the following steps: inoculating the recombinant strain onto a seed medium, culturing to obtain a seed liquid, transferring the seed liquid into a fermentation medium at an inoculation quantity of 20%, and culturing to produce L-threonine.

In some embodiments, the seed medium is: 5 g/L corn steep liquor powder, 20 g/L glucose, 5 g/L yeast powder, 2 g/L KH₂PO₄, 1 g/L magnesium sulfate, 20 mg/L FeSO₄·7H₂O, and 20 mg/L MnSO₄·H₂O; the fermentation medium is: 20 g/L glucose, 2 g/L potassium dihydrogen phosphate, 3 g/L yeast powder, 1 g/L betaine, 1 g/L magnesium sulfate, 10 mg/L FeSO₄·7H₂O, 10 mg/L MnSO₄·H₂O, 8 g/L corn steep liquor powder, and 10 mg/L vitamin B1.

Compared with the existing art, the present application has at least the following beneficial effects.
(1) The present application provides a mutant MntP manganese ion transporter which can increase the threonine yield of a strain when expressed in the strain. For example, after the THRS strain expresses the mutant MntP manganese ion transporter, the threonine yield of the recombinant strain is increased by 5.38%, and the threonine yield reaches 105.7 g/L after 48 hours of fermentation; after the THRS-6 strain expresses the mutant MntP manganese ion transporter, the threonine yield of the recombinant strain is increased by 3.91%, and the threonine yield reaches 130.2 g/L after 48 hours of fermentation.
(2) The mutant MntP manganese ion transporter can also combine with other mutations, thereby further increasing the threonine yield and providing a synergistic technical effect. For example, the THRS strain expresses MntP G25D and YbiI Q86L simultaneously, the threonine yield is increased by 15.15%, and the threonine yield reaches 115.5 g/L after 48 hours of fermentation; the THRS-6 strain expresses MntP G25D and YbiI Q86L simultaneously, the threonine yield is increased by 20.67%, and the threonine yield reaches 151.2 g/L after 48 hours of fermentation.

### DETAILED DESCRIPTION

To make the objects, technical solutions, and beneficial effects of the present application more apparent, the present application is further described in detail in conjunction with the embodiments. It should be understood that the specific examples described in the following embodiments of the present application are merely exemplary illustrations of the embodiments of the present application, are intended to explain the present application, and shall not be construed as limiting the present application.

Endpoints of the ranges and any values disclosed herein are not limited to the precise ranges or values, and such ranges or values should be understood as including values approximating to these ranges.

### Example 1: Construction of a THRS recombinant strain

### 1. Construction of an mntP gene knockout strain

The *mntP* gene in a THRS strain (Deposit No.: CGMCC No. 31694) was knocked out by homologous recombination. The specific steps are as follows:
Competent cells of *E*. *coli* were prepared. The pKD46 plasmid was induced with an arabinose stock solution and transferred into the competent cells of *E*. *coli*. The transferred cells were cultured and selected on an ampicillin-containing resistance medium. Homologous arms were selected at both ends of the target gene. Homology arm primers Pkan-F/Pkan-R to a kanamycin resistance gene *kan* were designed with the plasmid pKD13 as a template, and PCR amplification was carried out. The purified PCR product was transferred into a strain containing the pKD46 plasmid by electro-transformation to obtain a pKD46-kan strain. Homologous recombination occurred during the culture of the pKD46-kan strain at 30 °C to obtain a homologous recombinant strain. The pKD46 temperature-sensitive plasmid was eliminated by culturing the homologous recombinant strain at 37 °C. Resistance selection was carried out on a kanamycin-containing medium to obtain a strain with the target gene knocked out. PCR validation was carried out using a primer pair PmntP-F'/PmntP-R' to confirm whether the target gene had been successfully knocked out. Subsequently, the transferred pCP20 plasmid was introduced to express the flippase recombinase gene, thereby promoting homologous recombination at the FRT sites and finally achieving knockout of the *mntP* gene. The *mntP* gene knockout strain was cultured simultaneously on an LB medium and a kanamycin-containing medium. The strain that grew normally on the LB medium but did not grow on the resistance medium was identified as the *mntP* gene knockout strain. Finally, the pCP20 temperature-sensitive plasmid was eliminated by culturing the *mntP* gene knockout strain at 42 °C, and PCR amplification was carried out using a primer pair PpCP20-F/PpCP20-R for verification to obtain the *mntP* gene knockout strain (designated as the *THRS-*Δ*mntP* strain).

### 2. Construction of an mntP G25D gene mutant strain

Homology arm primers PmntP-F/PmntP-R were designed with the mutant *mntP G25D* sequence as a template, and a PCR amplification product of the exogenous *mntP G25D* gene was constructed. The PCR amplification product was transferred into *E. coli* THRS and replaced a target region via homologous recombination, thereby achieving knock-in of the *mntP G25D* mutant gene. The obtained recombinant strain was subjected to PCR validation and sequencing analysis. The sequencing result is shown in SEQ ID NO: 4, confirming successful obtainment of the *mntP G25D* gene mutant strain (designated as the *THRS-MntP G25D* mutant strain).

### 3. Construction of a ybiI Q86L gene mutant strain

Homology arm primers PybiI-F/PybiI-R (whose sequences were as shown in SEQ ID NO: 15 and SEQ ID NO: 16, respectively) were designed with the mutant ybiI sequence (SEQ ID NO: 14, designated as *ybiI Q86L*) as a template, and a PCR amplification product of the exogenous *ybiI Q86L* gene was constructed. The PCR amplification product was transferred into *E. coli* THRS and replaced a target region via homologous recombination. The specific implementation method was the same as the method in 1, and finally, knock-in of the *ybiI Q86L* mutant gene was achieved. The obtained recombinant strain was subjected to PCR validation and sequencing analysis. The sequencing result is shown in SEQ ID NO: 14, confirming successful obtainment of the *ybiI Q86L* gene mutant strain (designated as the THRS-YbiI Q86L mutant strain).

### 4. Construction of a mutant strain simultaneously containing mntP G25D and ybiI Q86L genes

Competent cells were prepared using another high-yield threonine-producing strain (*ybiI Q86L* gene mutant strain). A PCR amplification product of the exogenous *mntP G25D* gene was constructed using the homology arm primers PmntP-F/PmntP-R. The PCR amplification product was transferred into the competent cells of the *ybiI Q86L* gene mutant strain and replaced a target region via homologous recombination. The specific implementation method was the same as the method described above, and finally, knock-in of the *mntP G25D* mutant gene was achieved. The obtained recombinant strain was subjected to PCR validation and sequencing analysis. The sequencing result is shown in SEQ ID NO: 4, confirming the successful obtainment of the mutant strain simultaneously containing *mntP G25D* and *ybiI Q86L* genes (designated as the THRS-MntP G25D-YbiI Q86L mutant strain).

### Example 2: L-threonine fermentation production of a recombinant strain

The *THRS-YbiI Q86L* mutant strain, *THRS-*Δ*mntP* strain, *THRS-MntP G25D* mutant strain, and *THRS-MntP G25D-YbiI Q86L* mutant strain obtained in Example 1 and the original *THRS* strain were inoculated onto a seed medium and cultured to obtain a seed liquid, respectively. The seed medium included the following components at the indicated concentrations: 4.2 g/L corn steep liquor, 10 g/L glucose, 2.5 g/L yeast powder, 2 g/L KH₂PO₄, 1.2 g/L magnesium sulfate, 20 mg/L FeSO₄·7H₂O, 20 mg/L MnSO₄·H₂O, and 30 mg/L biotin. Three parallel replicates were set up for each set of experiments.

The seed culture obtained by the above culturing method was transferred into a fermentation medium at an inoculation quantity of 20% for culture.

### 1. Process control in a 5 L seed tank

a. The temperature was set to 37 °C, the pH was set to 7.0, the agitation speed was set to 500 rpm, and the aeration rate was set to 0.3 m³/h; the temperature was maintained at 37 °C throughout the process; the tank pressure was 0.05-0.08 MPa; the culture period was 10 hours.
b. Inoculation standard: OD₆₀₀: 12-15.
c. The seed medium consisted of 5 g/L corn steep liquor powder, 20 g/L glucose, 5 g/L yeast powder, 2 g/L KH₂PO₄, 1 g/L magnesium sulfate, 20 mg/L FeSO₄·7H₂O, and 20 mg/L MnSO₄·H₂O.

### 2. Process control for fermentation in a 5 L fermenter

a. The temperature was set to 37 °C, the pH was set to 7.0, the initial agitation speed was set to 300 rpm, and the aeration rate was set to 0.3 m³/h; the temperature was maintained at 37 °C throughout the process; the tank pressure was 0.05-0.08 MPa.
c. DO control: At 0 h, the aeration rate was 0.3 m³/h, theagitation speed was 300 rpm, and the tank pressure was 0.05 MPa.
d. When DO dropped to 30% or below, the dissolved oxygen level was controlled at 30% by adjusting the aeration rate and the stirring speed until the end of fermentation.
e. The fermentation medium consisted of 20 g/L glucose, 2 g/L potassium dihydrogen phosphate, 3 g/L yeast powder, 1 g/L betaine, 1 g/L magnesium sulfate, 10 mg/L FeSO₄·7H₂O, 10 mg/L MnSO₄·H₂O, 8 g/L corn steep liquor powder, and 10 mg/L vitamin B1.

### 3. Determination method for threonine

(1) Sample processing: 1 mL of fermentation broth obtained after 48 h of fermentation was taken and centrifuged at 12000 rpm for 10 min to remove bacterial cells, and the supernatant was collected. The supernatant was appropriately diluted with deionized water and filtered through a 0.22 µm membrane.
(2) Analytical method: OPA precolumn derivatization.
(3) Chromatographic conditions:
   1) Chromatographic column: C18 column (250 × 4.6) mm.
   2) Column temperature: 40 °C.
   3) Mobile phase A: 3.01 g of anhydrous sodium acetate was weighed into a beaker, dissolved with ultrapure water, and brought to a constant volume of 1 L. 200 µL of triethylamine was added. The pH was adjusted to 7.20 ± 0.05 with 5% acetic acid. After filtration, 5 mL of tetrahydrofuran was added, and the solution was mixed, filtered through a 0.22 µm inorganic membrane, and degassed in an ultrasonic bath for 20 min for later use.
      Mobile phase B: 3.01 g of anhydrous sodium acetate was weighed into a beaker, dissolved with ultrapure water, and brought to a constant volume of 200 mL. The pH was adjusted to 7.20 ± 0.05 with 5% acetic acid. 400 mL of acetonitrile and 400 mL of methanol were added to the solution, and the solution was mixed, filtered, and degassed in an ultrasonic bath for 20 min for later use.
   4) Flow rate: 1.0 mL/min.
   5) UV detection wavelength: 338 nm.
   6) Column temperature: 40 °C.

### 4. Validation of production performance of strains in 5 L fermenters

**Table 1 L-threonine production performance of strains in 5 L fermenters**

| Strain | Yield (g/L) | Conversion rate (%) |
|---|---|---|
| THRS strain | 100.3 g/L | 30.2% |
| THRS-YbiI Q86L mutant strain | 107.8 g/L | 29.7% |
| THRS-△*mntP* strain | 91.2 g/L | 29.3% |
| THRS-MntP G25D mutant strain | 105.7 g/L | 31.1% |
| THRS-MntP G25D-YbiI Q86L | 115.5g/L | 30.7% |

The results show that after glycine at position 25 of the amino acid sequence of the protein encoded by the *mntP* gene is replaced with aspartic acid, the L-threonine yield is significantly increased compared with the original strain. *MntP G25D* and *YbiI Q86L* can act synergistically, thereby further increasing the L-threonine yield. The THRS-MntP G25D-YbiI Q86L mutant strain is designated as FFTHR-38.

### Example 3: Construction of a THRS-6 recombinant strain and L-threonine production

According to the method in Example 1, a THRS-6-YbiI Q86L mutant strain, a THRS-6-Δ*mntP* strain, a THRS-6-MntP G25D mutant strain, and a THRS-6-MntP G25D-YbiI Q86L mutant strain were constructed based on the THRS-6 strain (Deposit No.: CGMCC No. 31695), respectively. Validation results of the production performance of the strains in the 5 L fermenters are shown in the table below.

**Table 2 L-threonine production performance of strains in 5 L fermenters**

| Strain | Yield (g/L) | Conversion rate (%) |
|---|---|---|
| THRS-6 | 125.3 g/L | 42% |
| THRS-6-YbiI Q86L | 142.1g/L | 44% |
| THRS-6-△*mntP* | 105.2 g/L | 29% |
| THRS-6-MntP G25D | 130.2 g/L | 43.3% |
| THRS-6-MntP G25D-YbiI Q86L | 151.2 g/L | 45.1% |

The results show that after the strain is replaced with the THRS-6 strain and glycine at position 25 of the amino acid sequence of the protein encoded by the *mntP* gene is replaced with aspartic acid, the L-threonine yield is significantly increased compared with the *THRS*-6 strain. Similarly, in the THRS-6 strain, *MntP G25D* and *YbiI Q86L* can act synergistically, thereby further increasing the L-threonine yield. It can be seen that the MntP G25D of the present application, when used in different strains, can increase the L-threonine yield and has a synergistic effect with YbiI Q86L. The THRS-6-MntP G25D-YbiI Q86L mutant strain is designated as FFTHR-39.

Nucleotide sequences and amino acid sequences involved in the present application are as follows:
SEQ ID NO: 1: (MntP protein) 
MNITATVLLAFGMSMDAFAASIGKGATLHKPKFSEALRTGLIFGAVETLTPLIGWGMGM
LASRFVLEWNHWIAFVLLIFLGGRMIIEGFRGADDEDEEPRRRHGFWLLVTTAIATSLDA
MAVGVGLAFLQVNIIATALAIGCATLIMSTLGMMVGRFIGSIIGKKAEILGGLVLIGIGVQI
LWTHFHG.
SEQ ID NO: 2: (mutant MntP protein) 
MNITATVLLAFGMSMDAFAASIGKDATLHKPKFSEALRTGLIFGAVETLTPLIGWGMGM
LASRFVLEWNHWIAFVLLIFLGGRMIIEGFRGADDEDEEPRRRHGFWLLVTTAIATSLDA
MAVGVGLAFLQVNIIATALAIGCATLIMSTLGMMVGRFIGSIIGKKAEILGGLVLIGIGVQI
LWTHFHG.
SEQ ID NO: 3: (*mntP* gene) 
atgaatatcactgctactgttcttcttgcgtttggtatgtcgatggatgcatttgctgcatcaatcggtaaaggtgccaccctccataaaccgaa
attttctgaagcattgcgaaccggccttattttggtgccgtcgaaaccctgacgccgctgatcggctggggaatgggcatgttagccagcc
ggtttgtccttgaatggaaccactggattgcgtttgtgctgctgatattcctcggcgggcgaatgattattgagggttttcgtggcgcagatgat
gaagatgaagagccgcgccgtcgacacggtttctggctactggtaaccaccgcgattgccaccagcctggatgccatggctgtgggtgtt
ggtcttgctttcctgcaggtcaacattatcgcgaccgcattggccattggttgtgcaaccttgattatgtcaacattagggatgatggttggtcg
ctttatcggctcaattattgggaaaaaagcggaaattctcggcgggctggtgctgatcggcatcggcgtccagatcctctggacgcacttcc
acggttaa.
SEQ ID NO: 4: (*mntP* mutant gene) 
atgaatatcactgctactgttcttcttgcgtttggtatgtcgatggatgcatttgctgcatcaatcggtaaagatgccaccctccataaaccgaa
attttctgaagcattgcgaaccggccttatttttggtgccgtcgaaaccctgacgccgctgatcggctggggaatgggcatgttagccagcc
ggtttgtccttgaatggaaccactggattgcgtttgtgctgctgatattcctcggcgggcgaatgattattgagggttttcgtggcgcagatgat
gaagatgaagagccgcgccgtcgacacggtttctggctactggtaaccaccgcgattgccaccagcctggatgccatggctgtgggtgtt
ggtcttgctttcctgcaggtcaacattatcgcgaccgcattggccattggttgtgcaaccttgattatgtcaacattagggatgatggttggtcg
ctttatcggctcaattattgggaaaaaagcggaaattctcggcgggctggtgctgatcggcatcggcgtccagatcctctggacgcacttcc
acggttaa.
SEQ ID NO: 5: (Pkan-F primer sequence 5'→3')
   CGATGATGTGTTTTATGGATACCCCGGTCAGGACATTGTCAGGCTGGAGCTGCTTC.
SEQ ID NO: 6: (Pkan-R primer sequence 5'→3')
   GCCAGACGGACTTTAATATTCACCTCTGGCAGCGTTCTTATCCGGGGATCCGTCGACC .
SEQ ID NO: 7: (PmntP-F primer sequence 5'→3') CGATGATGTGTTTATGGATACCCCGGTCAGGACATTGTCATGAATATCACTGCTACTG TTCTTCTTGC.
SEQ ID NO: 8: (PmntP-R primer sequence 5'→3') 
GCCAGACGGACTTTAATATTCACCTCTGGCAGCGTTCTTATTAACCGTGGAAGTGCGT
CC.
SEQ ID NO: 9: (PmntP-F' primer sequence 5'→3')
   TTATGGATACCCCGGTCAGGACATTGTC.
SEQ ID NO: 10: (PmntP-R' primer sequence 5'→3')
   GGACTTTAATATTCACCTCTGGCAGCGTTCTTA.
SEQ ID NO: 11: (PpCP20-F primer sequence 5'→3')
   ATGTCTGAATTAGTTGTTTTCAAAGCAAATGAAC.
SEQ ID NO: 12: (PpCP20-R primer sequence 5'→3')
   GATCCTTCCGTATTTAGCCAGTATGTTCT.
SEQ ID NO: 13: (mutant YbiI protein sequence) 
MASGWANDDAVNEQINSTIEDAIARARGEIPRGESLDECEECGAPIPQARREAIPGVRLCI
HCQQEKDLQKPAYTGYNRRGSKDSLLR.
SEQ ID NO: 14: (nucleotide sequence of mutant *ybiI* gene) 
ttaacgtaacaggctgtctttcgaacctctgcgattatatcctgtataagctggtttttgtaaatctttctcctgctgacaatgaatacataagcgc
acgccaggaatggcttcccgacgggcctgcgggatgggggcaccgcactcttcacattcatccaggctttcgccgcgcggaatttcaccc
cgagcgcgggcaatcgcatcttcaattgtactgttgatctgttcgttgacggcgtcatcgttagcccaaccggatgccat.
SEQ ID NO: 15: (PybiI-F primer sequence 5'→3') 
AAATACAAATTATACTCGCTATGCATCTCGCCAGTTGTAGTTAACGTAACAGGCTGTC
TTTCGAACCTC.
SEQ ID NO: 16: (PybiI-R primer sequence 5'→3')

CTACACTTAACTGTACAAGTATTGATATGGGGAGGTCGATATGGCATCCGGTTGGGCT
AAC.

Finally, it is to be noted that the above embodiments are merely used to explain the technical solutions of the present application and are not construed as limiting the content of the present application. Various simple variations can be made to the technical solutions of the present application without departing from the scope of the technical concept of the present application, including combinations of various technical features in any other suitable manners. These simple variations and combinations should be construed as content disclosed in the present application and are all within the protection scope of the present application.

## Claims

1. Use of a mutant MntP manganese ion transporter for increasing threonine yield in a threonine-producing strain, wherein relative to a wild-type MntP manganese ion transporter having a sequence as shown in SEQ ID NO: 1, the mutant MntP manganese ion transporter comprises at least the following mutation site: the amino acid at position 25 of the wild-type MntP manganese ion transporter is mutated from glycine to aspartic acid.

2. The use according to claim 1, wherein an amino acid sequence of the mutant MntP manganese ion transporter is as shown in SEQ ID NO: 2.

3. A high-yield L-threonine-producing recombinant strain expressing a mutant MntP manganese ion transporter, wherein relative to a wild-type MntP manganese ion transporter having a sequence as shown in SEQ ID NO: 1, the mutant MntP manganese ion transporter comprises at least the following mutation site: the amino acid at position 25 of the wild-type MntP manganese ion transporter is mutated from glycine to aspartic acid.

4. The recombinant strain according to claim 3, wherein an amino acid sequence of the mutant MntP manganese ion transporter is as shown in SEQ ID NO: 2.

5. The recombinant strain according to claim 3, wherein the recombinant strain is *Escherichia coli*.

6. The recombinant strain according to claim 5, wherein the recombinant strain is obtained by modification based on an *Escherichia coli* THRS strain, an *Escherichia coli* THRS-6 strain, an *Escherichia coli* THRS-YbiI Q86L mutant strain, or an *Escherichia coli* THRS-6-YbiI Q86L mutant strain;
wherein the THRS-YbiI Q86L mutant strain is an *Escherichia coli* THRS strain expressing a mutant YbiI protein, and the THRS-6-YbiI Q86L mutant strain is an *Escherichia coli* THRS-6 strain expressing a mutant YbiI protein, wherein an amino acid sequence of the mutant YbiI protein is as shown in SEQ ID NO: 13.

7. A method for preparing the recombinant strain according to any one of claims 3 to 6, comprising: designing homologous arm primers with a gene encoding a mutant MntP manganese ion transporter as a template, constructing a PCR amplification product, transferring the PCR amplification product into a threonine-producing strain, and replacing a target region via homologous recombination to obtain the recombinant strain.

8. The method according to claim 7, wherein the threonine-producing strain is *Escherichia coli*.

9. The method according to claim 7, wherein the threonine-producing strain is an *Escherichia coli* THRS strain, an *Escherichia coli* THRS-6 strain, an *Escherichia coli* THRS-YbiI Q86L mutant strain, or an *Escherichia coli* THRS-6-YbiI Q86L mutant strain;
wherein the THRS-YbiI Q86L mutant strain is an *Escherichia coli* THRS strain expressing a mutant YbiI protein, and the THRS-6-YbiI Q86L mutant strain is an *Escherichia coli* THRS-6 strain expressing a mutant YbiI protein, wherein an amino acid sequence of the mutant YbiI protein is as shown in SEQ ID NO: 13.

10. A method for producing L-threonine, comprising: inoculating the recombinant strain according to any one of claims 3 to 6 onto a seed medium, culturing to obtain a seed liquid, transferring the seed liquid into a fermentation medium at an inoculation quantity of 20%, and culturing to produce L-threonine.
